(19) Europäisches Patentamt

European Patent Office

Office européen des brevets

(11) Numéro de publication: **0 313 463 B1**

(12) **FASCICULE DE BREVET EUROPEEN**

(45) Date de publication de fascicule du brevet: **24.03.93** (51) Int. Cl.⁵: **C08J 3/12**, C08L 5/00

(21) Numéro de dépôt: **88402641.0**

(22) Date de dépôt: **20.10.88**

(54) **Compositions granulées de polysaccharides procédé pour leur préparation et utilisation.**

(30) Priorité: **21.10.87 FR 8714559**

(43) Date de publication de la demande:
**26.04.89 Bulletin 89/17**

(45) Mention de la délivrance du brevet:
**24.03.93 Bulletin 93/12**

(84) Etats contractants désignés:
**AT BE CH DE ES FR GB GR IT LI LU NL SE**

(56) Documents cités:
**EP-A- 0 256 538**
**FR-A- 2 586 030**
**US-A- 2 868 664**
**US-A- 4 517 216**

**JOURNAL OF PHARMACEUTICAL SCIENCES,
vol. 71, no. 6, juin 1982, pages 628-632, American Pharmaceutical Association; J.N. Mc-MULLEN et al.: "Pectin-gelatin complex coacervates I: Determinants of microglobule size, morphology, and recovery as water-dispersible powders"**

(73) Titulaire: **ELF SANOFI
32-34, rue Marbeuf
F-75008 Paris(FR)**

(72) Inventeur: **Ridoux, Claude
Chemin des Busclats
F-84800 Isle-sur-Sorgue(FR)**

(74) Mandataire: **Gillard, Marie-Louise et al
Cabinet Beau de Loménie 55, Rue d'Amsterdam
F-75008 Paris (FR)**

EP 0 313 463 B1

**Description**

La présente invention concerne des compositions de polysaccharides épaississants, à forte dispersibilité dans l'eau, constituées de particules de polysaccharides revêtues d'une gélatine hydrolysée.

On sait que les polysaccharides, qui sont utilisés comme épaississants et viscosifiants des milieux aqueux, sont difficiles à disperser dans l'eau : ils s'agglomèrent et forment des grumeaux. On doit donc, pour dissoudre une poudre de ces polysaccharides dans l'eau, ne les introduire que peu à peu en agitant violemment le milieu ou après avoir humecté préalablement la poudre ; d'autres moyens ont aussi été proposés pour faciliter la dissolution de ces poudres et, par exemple :

- le traitement du polysaccharide par des aldéhydes, par exemple par le formaldéhyde ou le glyoxal, ce qui améliore leur dispersibilité mais diminue la vitesse de dissolution des polymères ;
- l'addition au polysaccharide ou dans le milieu à viscosifier d'adjuvants de dissolution, comme le lignosulfonate de sodium ajouté au xanthane et scléroglucane selon le brevet FR-A-2577982, ou les sucres ajoutés en très forte proportion (8 à 10 fois) aux pectines ;
- la déshydration, en lit fluidisé, d'une solution du polysaccharide déposée sur un support divisé inerte, dont le polysaccharide anhydre se détache facilement, selon EP-A-206368,
- le mouillage préalable, notamment des pectines, par un alcool ou un polyol.

On a maintenant trouvé que l'on pouvait obtenir des compositions de polysaccharides épaississants et viscosifiants, mouillables et faciles à disperser en milieu aqueux en revêtant les particules de polysaccharide avec une gélatine hydrolysée, du moment que le dit polysaccharide n'était pas porteur de nombreux groupes anioniques.

On a décrit l'amélioration de la mouillabilité et de la dispersibilité de la gélatine par pelliculage avec une gélatine hydrolysée dans FR-A-2586030, mais on ne pouvait en conclure que la même solution pouvait être appliquée à certains polymères de nature et de propriétés très différentes.

Selon l'invention, la poudre de polysaccharide est traitée, par un procédé classique, dans un granulateur avec une solution aqueuse de la gélatine convenablement choisie. Ceci donne une composition de granulométrie supérieure à celle du produit de départ, et un autre avantage de l'invention est l'élimination des fines poussières de polysaccharide avec, notamment, une amélioration de la coulabilité du produit.

Les granulés, objet de l'invention, peuvent être préparés avec des polysaccharides viscosifiants et épaississants, ne portant pas de nombreux groupes anioniques, résultant soit d'une extraction de matériel végétal, comme les galactomannanes, les amidons et les pectines naturelles ou ses dérivés amidés, ou de la fermentation du glucose par des micro-organismes, comme le xanthane, le dextrane et le scléroglucane ou avec des mélanges de tels polysaccharides. L'amélioration de la mouillabilité et de la dispersibilité est particulièrement sensible dans le cas du xanthane et des galactomannanes : le guar et la caroube, et elle est très nette, même pour les pectines faiblement méthylées, par contre, elle est pratiquement nulle pour des polysaccharides, qui portent de nombreux groupes anioniques comme les alginates et les carraghénanes, salifiés ou non, et ceux-ci ne font donc pas partie de l'invention.

La gélatine hydrolysée, utilisée pour la granulation, est une gélatine de masse moléculaire, comprise entre 1 000 et 50 000 Daltons et de préférence entre 3 000 et 15 000 Daltons, résultant de l'hydrolyse par voie enzymatique ou chimique d'une gélatine obtenue de façon classique par hydrolyse du collagène des peaux et des os animaux ; cette gélatine hydrolysée est soluble dans l'eau à 20°C, n'a plus de force en gelée mesurable et ses solutions aqueuses à 20 % (p/v) ont une viscosité, mesurée à 25°C par la méthode de la pipette viscosimétrique à écoulement, comprise entre 1,5 mPa.s et 75 mPa.s ; cette méthode de mesure est décrite dans l'ouvrage "Standard methods for the sampling and testing of gelatins" édité par Gelatin Manufacturers Institute of America Inc. Ces qualités de gélatine hydrolysée se trouvent dans le commerce, ou peuvent être préparées en utilisant les procédés décrits notamment dans "Novo enzyme Information 1636-GB" à partir d'une gélatine de type A ou B provenant de l'hydrolyse du collagène des peaux et os de porcins et bovins.

Les granulés, objet de l'invention, contiendront en général de 0,5 à 5 % en poids de gélatine hydrolysée par rapport au poids du polysaccharide.

La granulation peut être effectuée dans un granulateur classique par empotage puis granulation, dans un granulateur à lit de gaz fluidisé ou dans un granulateur rapide, dans lequel on introduit le polysaccharide pulvérulent avec une solution de la gélatine hydrolysée, sous la forme d'une solution aqueuse ou d'une solution hydroalcoolique. Les conditions de la granulation dépendent des caractéristiques physicochimiques du polysaccharide et de la gélatine hydrolysée ; le flux d'air dans un lit à air fluidisé dépend évidemment de la taille et de la densité des particules.

Pour un galactomannane, dans un granulateur à lit d'air fluidisé, on pulvérisera une solution aqueuse de gélatine hydrolysée, contenant de 2 à 25 % en poids et de préférence de 5 à 10 % de gélatine hydrolysée, sur la poudre de polysaccharide en suspension dans un lit fluidisé, l'air étant introduit à une température comprise entre 20°C et 80°C et de préférence entre 40°C et 70°C.

Les compositions de polysaccharides granulées selon l'invention peuvent être utilisées dans toutes les applications classiques de polysaccharides en poudre ; elles sont particulièrement avantageuses pour la préparation de produits à dilution aqueuse, dite instantanée dans les domaines alimentaire ou pharmaceutique et un autre objet de l'invention est l'application de ces granulés à la préparation de compositions à dilution facile et rapide pour donner des suspensions aqueuses, stables à destination alimentaire ou pharmaceutique. Certains de ces polysaccharides granulés et notamment les galactomannanes, à concentration égale ou supérieure à 5 %, donnent même des gels, à formation quasi instantannée, par dispersion et gonflement immédiat dans une phase aqueuse.

Parmi les applications alimentaires, on peut citer les préparations instantanées pour crèmes, flans ou boissons chocolatées instantanées.

Dans le domaine pharmaceutique, l'introduction de granulés du polysaccharide dans un mélange pulvérulent de principe actif et d'excipients permet, lorsque le principe actif est insoluble dans l'eau, d'obtenir une formulation qui donne, pratiquement sans agitation, une suspension homogène stable du principe actif ; ils auront notamment leur application dans la préparation de suspensions buvables ou de crèmes, par exemple en pédiatrie.

Des granulés selon l'invention seront aussi avantageusement utilisés pour des applications techniques, par exemple dans des suspensions pour encollage ou dans les apprêts textiles.

Des exemples de mise en oeuvre de l'invention sont décrits dans ce qui suit.

Par définition, la dispersibilité est mesurée par le temps mis par la poudre pour se répartir dans l'ensemble liquide, après avoir été déposée sur la surface de l'eau ; pour déterminer cette valeur, on place 1 ou 2 g du produit à étudier dans un entonnoir dont la tige bouchée se trouve juste au-dessus de la surface de 100 ml d'eau qui ont été introduits dans un bécher de 250 ml et on mesure le temps mis par la poudre pour pénétrer dans le liquide et s'y disperser après le débouchage de la tige soit sans agitation, soit avec une agitation mécanique (750 tours/minute).

EXEMPLE 1

On fluidise dans un granulateur de type Uniglatt, commercialisé par la Société Glatt-France, 400 g de poudre de guar, dans un lit d'air à 65°C et pulvérise dans ce lit 200 ml d'une solution dont la température est voisine de 20°C et qui contient 10 % (p/v) de gélatine hydrolysée à un débit de 10 ml/min et sous une pression de 5 x 10⁴ Pa.

On maintient les granulés dans le lit fluidisé durant 5 min après la fin de la pulvérisation pour les sécher.

La gélatine hydrolysée mise en oeuvre est une gélatine atomisée, hydrosoluble à 20°C, de masse moléculaire 6 000 Daltons, obtenue par hydrolyse enzymatique d'une gélatine de type B.

Avec une gomme guar, comme celle commercialisée par la Société Mero Rousselot Satia (France) sous la référence Viscogum$^R$ HV 3000 A, ayant les caractéristiques suivantes, :

| | |
|---|---|
| - densité apparente<br>- volume apparent | 0,727 g/ml<br>1,375 ml/g |
| - dispersibilité de 2 g sans agitation | |
| à 20°C<br>à 60°C<br>- granulométrie | 8 heures<br>3 heures 36 min<br>87 % traverse le tamis de 200 mesh |

on obtient des granulés contenant 5 % de gélatine caractérisés par les valeurs suivantes :

| - densité apparente<br>- volume apparent | 0,251 g/ml<br>3,975 ml/g |
|---|---|
| - dispersibilité de 2 g sans agitation | |
| à 20°C<br>à 60°C<br>- granulométrie | 9 secondes<br>3 secondes<br>31,45 % traverse le tamis de 200 mesh |

EXEMPLE 2

En appliquant la méthode indiquée à l'exemple 1, on granule 400 g de poudre de xanthane avec 400 ml d'une solution à 5 % de la même gélatine hydrolysée, qui est introduite dans le granulateur sous une pression de $10^5$ Pa à un débit de 100 ml/min.

Durant la granulation, l'air est introduit à 65°C et, à la fin de la pulvérisation, on sèche les granulés pendant 10 min, avec de l'air à 60°C. Les caractéristiques de la poudre de xanthane de départ et des granulés préparés sont mentionnées dans le tableau I suivant :

TABLEAU I

| Caractéristiques | Poudre | Granulés |
|---|---|---|
| - densité apparente g/ml | 0,689 | 0,262 |
| - volume apparent ml/g | 1,45 | 3,815 |
| - dispersibilité de 2 g, à 20°C, sans agitation | 1 h 25 | 27 min |
| - dilution dans l'eau sous agitation à 20°C | Grumeaux non mouillable | pas de grumeaux |

EXEMPLE 3

On granule un mélange homogène de 200 g de la poudre de xanthane mise en oeuvre à l'exemple précédent avec 200 g de poudre de caroube en pulvérisant 200 ml de solution à 6 % de la gélatine hydrolysée utilisée à l'exemple 1, sous une pression de 7 x $10^4$ Pa et avec un débit de 15 ml/min ; l'air est introduit dans le granulateur à 80°C ; en fin de granulation, on sèche 10 min.

Les granulés contiennent 3 % en poids de gélatine par rapport au poids du polysaccharide.

Dans un autre essai, on introduit 350 ml de la même solution de gélatine hydrolysée pour obtenir des granulés contenant 5 % de celle-ci, au lieu de 3 %.

Dans le tableau II, sont portées les caractéristiques du mélange de poudres de départ et celles des granulés obtenus ; le granulé à 5 % est réellement instantanéisé.

TABLEAU II

| Caractéristiques | Poudre | Granulé à 3 % | Granulé à 5 % |
|---|---|---|---|
| densité apparente g/ml | 0,735 | 0,575 | 0,273 |
| volume apparent ml/g | 1,36 | 2,74 | 3,66 |
| dispersibilité de 2 g, à 20°C sans agitation | 15 min avec gros grumeaux | 3 min 30 avec quelques grumeaux | 10 s homogène |
| dispersibilité de 1 g à 20°C avec agitation | 5 s avec gros grumeaux | 5 s homogène | 5 s homogène |

EXEMPLES 4 ET 5

Dans un lit d'air fluidisé à 45°C, on introduit 300 g de pectine fortement méthylée (HM) en poudre, ou d'une pectine faiblement méthylée (LM) mais amidée, telle que celle commercialisée sous la référence Unipectine 325 NH 95 par Sanofi Bio Industrie (Fr), et on y pulvérise, à un débit de 10 ml/min et sous une pression de 5 x $10^4$ Pa, 180 ml d'une solution hydroalcoolique ($H_2O/C_2H_5OH$-50/50-V/V) de gélatine hydrolysée à 5 % (P/V).

On maintient les granulés dans le lit fluidisé pendant 10 min après la fin de la pulvérisation.

Dans le tableau III, on a porté les caractéristiques des poudres de départ et des granulés de l'invention.

## TABLEAU III

| Caractéristiques | Poudre de départ | | Granulés | |
| --- | --- | --- | --- | --- |
| | Pectine HM | Pectine LM amide | Pectine HM | Pectine LM amide |
| densité apparente g/ml | 0,671 | 0,727 | 0,234 | 0,395 |
| vol. apparent ml/g dispersibilité de 2 g* : | 1,49 | 1,375 | 4,28 | 2,53 |
| - à 20°C | grumeaux non mouillable | lentille en surface | 11 s (gel en 1 h) | immédiat (gel en 45 min) |
| - à 60°C | grumeaux non mouillable | gros grumeaux | 3 s (gel en 10 min) | immédiat (gel en quelques minutes) |

\* avec agitation manuelle pour la poudre de départ, sans agitation pour les granulés.

EXEMPLE 6

On granule avec une solution aqueuse de gélatine hydrolysée, comme décrit dans l'exemple 4, pour obtenir des granulés à 3 % de gélatine (P/P) une pectine LM non amidée.

Les résultats figurent dans le tableau IV.

TABLEAU IV

| Caractéristiques | Poudre de départ | Granulés |
|---|---|---|
| densité apparente g/ml | 0,690 | 0,292 |
| volume apparent (ml/g) dispersibilité de 2 g* : | 1,45 | 3,43 |
| - à 20°C | grumeaux | 9 s (gel en 45 min) |
| - à 60°C | motte non mouillable | 3 s (gel en 10 min) |

* sans agitation pour les granulés ; avec agitation mannuelle pour la poudre.

**Revendications**

1. Compositions granulées de polysaccharides viscosifiants et épaississants ne portant pas de nombreux groupes anioniques choisis parmi les galactomannanes, les amidons, les pectines naturelles ou leurs dérivés amidés, le xanthane, le dextrane, le scléroglucane et leurs mélanges, caractérisées en ce que les particules desdits polysaccharides sont revêtues de gélatine hydrolysée ayant une masse moléculaire comprise entre 1 000 et 50 000 Daltons, à raison de 0,5 % à 5 % en poids de gélatine hydrolysée.

2. Compositions selon la revendication 1, caractérisées en ce que ladite gélatine hydrolysée est soluble dans l'eau à 20°C et a une force en gelée non-mesurable.

3. Compositions selon l'une des revendications 1 ou 2, caractérisées en ce que le polysaccharide est un xanthane.

4. Compositions selon l'une des revendications 1 ou 2, caractérisées en ce que le polysaccharide est un galactomannane.

5. Compositions selon l'une des revendications 1 ou 2, caractérisées en ce que le polysaccharide est une pectine naturelle ou amidée.

6. Compositions selon l'une des revendications 1 ou 2, caractérisées en ce que le polysaccharide est un scléroglucane.

7. Compositions selon l'une quelconque des revendications 1 à 6, caractérisées en ce que le polysaccharide est un mélange de polysaccharides.

8. Procédé de préparation des granulés de l'une des revendications 1 à 7, caractérisé en ce que les particules de polysaccharide sont granulées en présence d'une solution de gélatine hydrolysée.

9. Utilisation des compositions selon l'une quelconque des revendications 1 à 7 pour la préparation de compositions pharmaceutiques, alimentaires ou techniques à dilution aqueuse rapide.

**Claims**

1. Granulated compositions of thickening and viscosity-raising polysaccharides not bearing numerous anionic groups, selected from the galactomannans, starch, natural pectins or their amido derivatives, xanthan, dextran, scleroglucan and mixtures thereof, characterized in that the polysaccharide particles are coated with hydrolysed gelatin having a molecular weight ranging between 1000 and 50 000 daltons, in the proportion of 0.5 to 5.0% by weight of hydrolysed gelatin.

2. Compositions according to claim 1, characterized in that said hydrolysed gelatin is soluble in water at 20°C and has a non-measurable gel power.

3. Compositions according to one of claims 1 or 2, characterized in that the polysaccharide is a xanthan.

**4.** Compositions according to one of claims 1 or 2, characterized in that the polysaccharide is a galactomannan.

**5.** Compositions according to one of claims 1 or 2, characterized in that the polysaccharide is a natural or amido pectin.

**6.** Compositions according to one of claims 1 or 2, characterized in that the polysaccharide is a scleroglucan.

**7.** Compositions according to any one of the claims 1 to 6, characterized in that the polysaccharide is a mixture of polysaccharides.

**8.** Process for the preparation of the granulates of one of claims 1 to 7, characterized in that the polysaccharide particles are granulated in the presence of a solution of hydrolysed gelatin.

**9.** Use of the compositions according to any one of claims 1 to 7 for the preparation of pharmaceutical, dietary or technical compositions having instant aqueous dilution properties.

**Patentansprüche**

**1.** Gekörnte, die viskosifizierende und verdickende Polysaccharidzusammensetzungen, die nicht zahlreiche anionische Gruppe enthalten, die ausgewählt werden unter Galactomannanen, Stärken, natürlichen Pektinen oder ihren amidierten Derivaten, Xanthan, Dextran, Scleroglucan und ihren Gemischen, dadurch **gekennzeichnet,** daß
die Polysaccharidteilchen mit hydrolysierter Gelatine einer Molekülmasse von zwischen 1000 und 50000 Dalton in einer Menge von 0,5 bis 5 Masse-% hydrolysierter Gelatine verkleidet sind.

**2.** Zusammensetzungen nach Anspruch 1,
dadurch gekennzeichnet, daß
die hydrolysierte Gelatine in Wasser von 20 ° C löslich ist und eine nicht meßbare Gelkraft besitzt.

**3.** Zusammensetzungen nach einem der Ansprüche 1 oder 2,
dadurch gekennzeichnet, daß
das Polysaccharid ein Xanthan ist.

**4.** Zusammensetzungen nach einem der Ansprüche 1 oder 2,
dadurch gekennzeichnet, daß
das Polysaccharid eine Galactomannan ist.

**5.** Zusammensetzungen nach einem der Ansprüche 1 oder 2,
dadurch gekennzeichnet, daß
das Polysaccharid ein natürliches oder amidiertes Pektin ist.

**6.** Zusammensetzungen nach einem der Ansprüche 1 oder 2,
dadurch gekennzeichnet, daß
das Polysaccharid ein Scleroglucan ist.

**7.** Zusammensetzungen nach einem der Ansprüche 1 bis 6,
dadurch gekennzeichnet, daß
das Polysaccharid ein Gemisch von Polysacchariden ist.

**8.** Verfahren zur Herstellung von Körnern nach einem der Ansprüche 1 bis 7,
dadurch gekennzeichnet, daß
die Polysaccharidteilchen in Gegenwart einer Lösung hydrolysierter Gelatine granuliert werden.

**9.** Verwendung von Zusammensetzungen nach einem der Ansprüche 1 bis 7 zur Herstellung pharmazeutischer, Nahrungsmittel- oder technischen Zusammensetzungen mit schneller Auflösung in Wasser.